# EUROPEAN PATENT APPLICATION

(11) **EP 4 755 979 A1**
(43) Date of publication of application: **10.06.2026**
(21) Application number: 24848841.3
(22) Date of filing: 09.07.2024
(51) Int. Cl.: C09C 3/06, A61K 8/19, A61K 8/25, A61K 8/26, A61K 8/27, A61K 8/28, A61K 8/29, A61Q 1/14, C03C 17/23, C03C 17/34, C03C 17/36, C09D 7/62, C09D 201/00

(54) **FLAKE-SHAPED BASE MATERIAL WITH WATER-REPELLENT FILM, COMPOSITION, COSMETIC PREPARATION, AND COATED BODY**

(30) Priority: 31.07.2023 JP 2023124760
(71) Applicant: NIPPON SHEET GLASS COMPANY, LIMITED, Tokyo 108-6321 (JP)
(72) Inventor: IWAI, Nobuki, Tokyo 108-6321 (JP); HORIGUCHI, Haruko, Tokyo 108-6321 (JP); MIKAMI, Shinji, Tokyo 108-6321 (JP)
(74) Representative: Cabinet Beau de Loménie
(86) International application number: PCT/JP2024/024755
(87) International publication number: WO 2025/028182

(57) **Abstract**

The present disclosure provides a flaky-shaped substrate with a water-repellent film 11 including a flaky substrate 1 and a water-repellent film 2 formed on the flaky substrate 1, the water-repellent film 2 including a rare-earth oxide.

## Description

### TECHNICAL FIELD

The present invention relates to a flaky-shaped substrate with a water-repellent film, and further relates to a composition, a cosmetic preparation, and a painted body each including a flaky-shaped substrate with a water-repellent film.

### BACKGROUND ART

Coated flaky substrates are added to compositions, such as cosmetic preparations and resin compositions, as needed. Coated flaky substrates are sometimes added to paint films on supports. Coated flaky substrates are added, for example, as pigments or as modifying agents for adjusting properties, such as elastic modulus.

In some cases, water repellency is required of coatings of flaky substrates. An example of such cases is a cosmetic preparation including an emulsion of oil and water, where a coated flaky substrate needs to be added to the oil. Silicone-containing films including a silicone-based material typified by dimethicone are known as water-repellent coatings. For example, Patent Literature 1 discloses a flaky substrate including an outermost layer formed of an acrylic silicone.

### CITATION LIST

### Patent Literature

Patent Literature 1: WO 2012/096182 A1

### SUMMARY OF INVENTION

### Technical Problem

It has been pointed out that silicone-based materials pose a risk to, for example, the environment. Hence, the present invention aims to provide a new flaky-shaped substrate having water repellency imparted by a non-silicone-based material.

### Solution to Problem

The present invention provides a flaky-shaped substrate with a water-repellent film: including a flaky substrate; and a water-repellent film formed on the flaky substrate, wherein
the water-repellent film includes a rare-earth oxide.

### Advantageous Effects of Invention

The present invention provides a new flaky-shaped substrate with a water-repellent film having water repellency imparted by a rare-earth oxide.

### BRIEF DESCRIPTION OF DRAWINGS

FIG. 1 is a cross-sectional view schematically showing an example of a flaky-shaped substrate with a water-repellent film.
FIG. 2 is a cross-sectional view schematically showing another example of a flaky-shaped substrate with a water-repellent film.
FIG. 3 is a perspective view showing an example of a flaky substrate.
FIG. 4 is a schematic diagram showing an exemplary apparatus for producing glass flakes.
FIG. 5 is schematic diagram showing another exemplary apparatus for producing glass flakes.

### DESCRIPTION OF EMBODIMENTS

Hereinafter, embodiments of the present invention will be described. The following description is not intended to limit the present invention to specific embodiments. Herein, the phrase "substantially coated" means that 80% or more, 85% or more, or even 90% or more of the surface area is coated. The phrase "consist substantially of" a certain component means that the component accounts for 80% or more, 85% or more, even 90% or more, or, in some cases, 95% or more of the total content on a mass basis. The term "main component" refers to a component with the highest content on a mass basis. The term "room temperature" refers to 20°C or higher and 30°C or lower.

The present invention is embodied, for example, according to the following aspects.

A flaky-shaped substrate with a water-repellent film according to a first aspect includes a flaky substrate and a water-repellent film formed on the flaky substrate, wherein
the water-repellent film includes a rare-earth oxide.

According to a second aspect, the flaky-shaped substrate with the water-repellent film according to the first aspect has enough water repellency to float on water at room temperature for three weeks.

According to a third aspect, in the flaky-shaped substrate with the water-repellent film according to the first or second aspect, the rare-earth oxide includes at least one selected from the group consisting of cerium oxide, lanthanum oxide, yttrium oxide, praseodymium oxide, and erbium oxide.

According to a fourth aspect, in the flaky-shaped substrate with the water-repellent film according to any one of the first to third aspects, the water-repellent film further includes at least one selected from the group consisting of aluminum oxide, zirconium oxide, titanium oxide, zinc oxide, and silicon oxide.

According to a fifth aspect, in the flaky-shaped substrate with the water-repellent film according to any one of the first to fourth aspects, the water-repellent film consists substantially of an inorganic substance.

According to a sixth aspect, in the flaky-shaped substrate with the water-repellent film according to any one of the first to fifth aspects, the flaky substrate consists substantially of an inorganic substance.

According to a seventh aspect, in the flaky-shaped substrate with the water-repellent film according to any one of the first to sixth aspects, the water-repellent film includes the rare-earth oxide as a main component.

According to an eighth aspect, the flaky-shaped substrate with the water-repellent film according to any one of the first to seventh aspects further includes an intermediate film between the flaky substrate and the water-repellent film.

A composition according to a ninth aspect includes the flaky-shaped substrate with the water-repellent film according to any one of the first to eighth aspects and a resin.

A cosmetic preparation according to a tenth aspect includes the flaky-shaped substrate with the water-repellent film according to any one of the first to eighth aspects.

A painted body according to an eleventh aspect includes: a paint film including the flaky-shaped substrate with the water-repellent film according to any one of the first to eighth aspects; and a support supporting the paint film.

### <Flaky-Shaped Substrate with Water-Repellent Film>

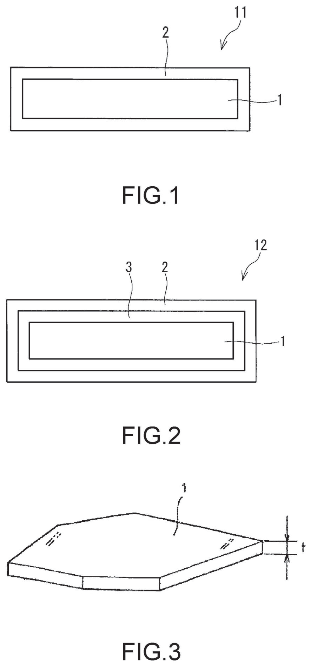

FIG. 1 shows a cross-section of an example of the flaky-shaped substrate with the water-repellent film. A flaky-shaped substrate with a water-repellent film (water-repellent film-attached flaky substrate) 11 includes a flaky substrate 1 and a water-repellent film 2 on the flaky substrate 1. The water-repellent film 2 is formed directly on the flaky substrate 1, and is in contact with a surface of the flaky substrate 1.

FIG. 2 shows a cross-section of another example of the flaky-shaped substrate with the water-repellent film. A flaky-shaped substrate with a water-repellent film 12 includes a flaky substrate 1, an intermediate film 3 on the flaky substrate 1, and a water-repellent film 2 on the intermediate film 3. The intermediate film 3 is formed between the flaky substrate 1 and the water-repellent film 2, and is in contact with the flaky substrate 1 and the water-repellent film 2.

The flaky substrate 1 may be substantially coated with the water-repellent film 2. If a proportion of the water-repellent film 2 coating the flaky substrate is low, the flaky-shaped substrate with the water-repellent film cannot exhibit a sufficient water repellency. The degree of the water repellency can be evaluated by a float test described later. As shown in FIGS. 1 and 2, the water-repellent film 2 may form the outermost layer.

### (Flaky Substrate)

The flaky substrate is a fine, plate-like thin piece and is also called, for example, a scaly substrate. FIG. 3 shows an example of the flaky substrate 1 having a thickness t. The flaky substrate is, for example, a glass flake, an alumina flake, mica, talc, or sericite. The flaky substrate is preferably a glass flake, an alumina flake, or mica. The mica may be natural mica or synthetic mica. Every material mentioned above as examples of the material of the flaky substrate is an inorganic substance. Any of the above materials may be used to form the flaky substrate consisting substantially of an inorganic substance.

A preferable average thickness of the flaky substrates is 10.0 µm or less, 5.0 µm or less, 3.0 µm or less, even 1.0 µm or less, or, in some cases, 0.8 µm or less. The lower limit of the average thickness is, for example, but not particularly limited to, 0.1 µm or more. The average thickness of the flaky substrates can be determined as the average of thicknesses of at least 50 flaky substrates. A thickness of each flaky substrate can be measured by observation using a scanning electron microscope (SEM).

A preferable average particle diameter of the flaky substrates is 200 µm or less, 100 µm or less, 50 µm or less, or even 30 µm or less. The lower limit of the average particle diameter of the flaky substrates is not limited to a particular value, and is, for example, 3 µm or more, or, in some cases, 5 µm or more. The average particle diameter of the flaky substrates can be determined as a particle diameter (D50) at 50% by cumulative volume from the smaller end of a particle size distribution of light scattering equivalent particle diameters measured by laser diffractometry.

A preferable aspect ratio of the flaky substrates is 10 or more, 15 or more, even 20 or more, or particularly 30 or more. The upper limit of the aspect ratio is not limited to a particular value, and may be 300 or less. The aspect ratio of the flaky substrates can be determined by dividing the average particle diameter by the average thickness.

The flaky substrate is particularly preferably a glass flake. The glass flake has a flat and smooth principal surface. Using the flat and smooth substrate is advantageous in terms of improving the later-described smoothness. A glass composition of the glass flake is not limited to a particular one. For example, a glass composition including silicon oxide as its main component and further including other metal oxides such as aluminum oxide, calcium oxide, and sodium oxide can be used. Specific examples of the glass composition include soda-lime glass, A-glass, C-glass, E-glass, ECR glass, borosilicate glass, and aluminosilicate glass.

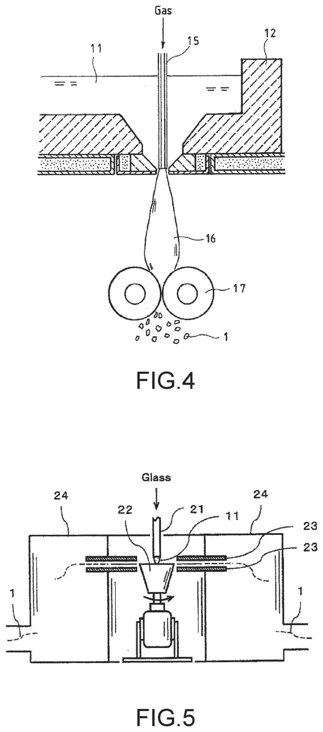

FIG. 4 shows an example of an apparatus for producing glass flakes by a blow process. The production apparatus is equipped with a refractory tank furnace 12, a blowing nozzle 15, and pressing rolls 17. A glass raw material 11 is melted in the refractory tank furnace 12 (melting furnace) and is inflated into a balloon by a gas delivered through the blowing nozzle 15, so that hollow glass 16 is obtained. The hollow glass 16 is crushed by the pressing rolls 17 to obtain a glass flake 1. The thickness of the glass flake 1 can be controlled by adjusting, for example, the speed of pulling the hollow glass 16 and the flow rate of the gas delivered through the blowing nozzle 15. The particle diameter of the glass flake 1 can be controlled by adjusting, for example, shaping, crushing, and classification conditions for the glass flake.

FIG. 5 shows an example of an apparatus for producing glass flakes by a rotary process. The apparatus is equipped with a rotary cup 22, a pair of annular plates 23, and an annular cyclone collector 24. A molten glass raw material 11 is poured into the rotary cup 22, centrifugally flows out from the upper edge of the rotary cup 22 in a radial manner, and is then drawn and carried into the annular cyclone collector 24 through the gap between the annular plates 23 by airflow. While passing through the annular plates 23, the glass is cooled and solidified into a thin film, which is then crushed into fine pieces to give a glass flake 1. The thickness of the glass flake 1 can be controlled by adjusting, for example, the distance between the annular plates 23 and the velocity of the airflow. The particle diameter of the glass flake 1 can be controlled by adjusting, for example, shaping, crushing, and classification conditions for the glass flake.

### (Water-Repellent Film)

The water-repellent film includes a rare-earth oxide, i.e., an oxide of a rare-earth element. Rare-earth elements include scandium, yttrium, and lanthanoids. The lanthanoids are 15 elements from lanthanum to lutetium (lanthanum, cerium, praseodymium, neodymium, promethium, samarium, europium, gadolinium, terbium, dysprosium, holmium, erbium, thulium, ytterbium, and lutetium) in the periodic table. The rare-earth oxide may be, for example, at least one selected from the group consisting of cerium oxide, lanthanum oxide, yttrium oxide, praseodymium oxide, and erbium oxide. The rare-earth oxide can impart water repellency to the flaky substrate.

The water-repellent film may consist substantially of an inorganic substance. The inorganic substance is not limited to a particular one, and may be, for example, at least one selected from the group consisting of an oxide, an oxynitride, and a nitride, or may include an oxide. The water-repellent film may consist substantially of an oxide. The water-repellent film may be free of silicone-based materials typified by a silicone oil. The water-repellent film may be free of fluorine-containing organic compounds.

The oxide is preferably, for example, cerium oxide. The water-repellent film may include cerium oxide. The water-repellent film may consist substantially of cerium oxide. The water-repellent film may consist substantially of the rare-earth oxide. However, the water-repellent film is not limited to this, and can include an oxide other than the rare-earth oxide.

The oxide other than the rare-earth oxide may include, for example, at least one selected from the group consisting of aluminum oxide, zirconium oxide, titanium oxide, zinc oxide, and silicon oxide. The oxide other than the rare-earth oxide may include at least one selected from the group consisting of aluminum oxide, zirconium oxide, and titanium oxide, or may include aluminum oxide and/or titanium oxide. These oxides can improve adhesiveness of the water-repellent film to the flaky substrate and thereby increase the durability of the water repellency.

The main component of the water-repellent film may be the rare-earth oxide. A content R1 of the rare-earth oxide in the water-repellent film may be, for example, 50% or more, 60% or more, or even 70% or more on a mass basis. A content R2 of the oxide other than the rare-earth oxide in the water-repellent film may be, for example, 5% or more, 10% or more, or even 15% or more on a mass basis. A ratio of M1 to M2 may exceed 1 (M1/M2 > 1), where M1 is a content of the rare-earth oxide on a molar basis and M2 is a content of the oxide other than the rare-earth oxide on a molar basis. M1/M2 may be 2 or more, 2.5 or more, or even 3 or more. M1/M2 may be, for example, 10 or less.

A film thickness of the water-repellent film may be, for example, 0.001 µm or more, 0.01 µm or more, or even 0.02 µm or more. The upper limit of the film thickness is, for example, but not particularly limited to, 1 µm or less.

### (Intermediate Film)

The intermediate film is an optional film that may be formed depending on a use, required properties, and the like of the flaky substrate. The intermediate film is provided, for example, to impart a desired color tone and/or a desired light reflectance to the flaky-shaped substrate with the water-repellent film. The light reflectance is, for example, an ultraviolet reflectance and an infrared reflectance. The intermediate film may be formed for another purpose, such as restriction of transfer of a substance from the flaky substrate to the water-repellent film. Transfer of a substance is, for example, diffusion of an alkaline component included in the flaky substrate to the water-repellent film. The intermediate film may be a single-layer film, or may be a multilayer film formed of two or more layers.

The intermediate film may be an optical interference film. The optical interference film can change the color tone and/or the reflectance of the flaky-shaped substrate with the water-repellent film by an optical interference effect involving the substrate and the water-repellent film each adjacent to the optical interference film. The flaky-shaped substrate with the water-repellent film including the optical interference film is suitable as a pigment, or is suitable as a shielding material for at least one selected from the group consisting of ultraviolet light and infrared light.

The configuration and the thickness of the intermediate film may be set as appropriate depending on the purpose of the intermediate film. The material of the intermediate film is not limited to a particular material, either. The intermediate film can include a known material, such as any of a variety of metals, oxides, oxynitrides, nitrides, and carbides. The intermediate film may consist substantially of an inorganic substance. Among the materials that can be included in the intermediate film, examples of the oxide are as follows. The intermediate film can include, for example, at least one selected from the group consisting of titanium oxide, silicon oxide, aluminum oxide, iron oxide, zinc oxide, tin oxide, zirconium oxide, cerium oxide, nickel oxide, chromium oxide, and vanadium oxide.

The material of the intermediate film is typically titanium oxide. The intermediate film may include titanium oxide as its main component. Titanium oxide has three crystalline forms, namely, anatase, brookite, and rutile, and anatase titanium oxide and rutile titanium oxide are industrially mass-produced. A preferred crystalline form of titanium oxide is rutile. Rutile titanium oxide has low photocatalytic activity and the highest refractive index.

Formation of a rutile titanium oxide film on the flaky substrate may be carried out according to a method disclosed, for example, in JP 2001-031421 A or JP 2003-012962 A. In the method disclosed in these patent publications, rutile titanium oxide precipitates on a glass flake in a solution containing a titanium compound such as titanium tetrachloride, forming a film on the glass flake. More specifically, the precipitation of rutile titanium oxide on the glass flake can be caused by adding an alkaline compound or an alkaline solution to the titanium compound-containing solution having a temperature of 55 to 85°C and a pH of 1.3 or less. Preliminarily depositing tin or a tin compound on the glass flake facilitates the precipitation of rutile titanium oxide. With the use of this method, a rutile titanium oxide film can be formed without the need for heating for crystal transition.

### (Other Constituent Members)

The flaky-shaped substrate with the water-repellent film is basically formed of the flaky substrate, the water-repellent film, and the intermediate film that is formed as necessary. However, the flaky-shaped substrate with the water-repellent film can include a constituent element other than these. For example, the flaky-shaped substrate with the water-repellent film may further include a plurality of particles deposited on the surface of the water-repellent film. The particles may include metal particles and/or inorganic pigment particles. The metal particles are, for example, fine gold particles.

### <Properties>

### (Water Repellency)

The flaky-shaped substrate with the water-repellent film according to the present embodiment can have a sufficient water repellency. The water repellency can be evaluated by a float test in water. That is, the water-repellent film having high water repellency can impart enough water repellency to the flaky substrate, which sinks in water on its own, to float on water for a long period of time. It is appropriate that the float test is performed over a long period of time, for example, three weeks or more, one month or more, or, in some cases, two months or more to measure the durability of the water repellency.

The float test can be performed by putting the flaky-shaped substrate with the water-repellent film into water at room temperature. The water may be prepared in a sufficient amount, for example, an amount 5 to 50 times the mass of the flaky-shaped substrate with the ware-repellent film. The water is left to stand still during the float test. A test specimen that has not sunk to the bottom of the container of the water and floating on the water surface or in the water at the end of the test period (e.g., three weeks) can be considered to have a sufficient water repellency.

The float test can also be used for sorting the flaky-shaped substrates. This is because the water-repellent film and the substrate are prone to be detached depending on their types, and water may enter through a portion partially lacking the water-repellent film to impair the water repellency. The float test may be performed as an inspection step for excluding such a defective piece. That is, the flaky-shaped substrates with the water-repellent film having the sufficient water repellency can be obtained by a manufacturing method including: manufacturing a plurality of flaky-shaped substrates with the water-repellent film; and putting the flaky-shaped substrates with the water-repellent film into water at room temperature, from which only those flaky-shaped substrates with the water-repellent film on the surface or in the water are collected after the water is left to stand still for three weeks.

### (Smoothness)

Unexpectedly, the flaky-shaped substrate with the water-repellent film according to the present embodiment can have a sufficient smoothness. The flaky substrate having a sufficient smoothness is particularly suitable for being included in cosmetic preparations. The smoothness can be evaluated using MIU and MMD as measures. MIU is an average of friction coefficients, while MMD is an average deviation of friction coefficients. MIU represents non-slipperiness; a smaller MIU value indicates higher smoothness. MMD represents roughness; a smaller MMD value indicates a smoother touch. The MIU value of the flaky-shaped substrate with the water-repellent film is, for example, 4 or less, 3 or less, or even 2 or less. The lower limit of the MIU value is, for example, but not particularly limited to, 0.2 or more. The MMD value of the flaky-shaped substrate with the water-repellent film is, for example, 0.8 or less, 0.6 or less, or even 0.4 or less. The lower limit of the MMD value is, for example, but not particularly limited to, 0.1 or more. Specific methods for measuring MIU and MMD will be described below.

### <Formation of Water-Repellent Film>

In production of the flaky-shaped substrate with the water-repellent film according to the present embodiment, the water-repellent film can be formed by a liquid-phase method. The flaky substrate with the water-repellent film formed by the liquid-phase method is dried and then heated. The heating is preferably performed at a temperature at which the water-repellent film can maintain the sufficient water repellency. The heating temperature is, for example, 400°C or higher, even 500°C or higher, or, in some cases, 600°C or higher. The upper limit of the heating temperature is not limited to a particular temperature, and the heating temperature may be 800°C or lower. The heating time is also preferably set to a time length during which the water-repellent film can maintain the sufficient water repellency. The heating time may be set as appropriate depending on, for example, the temperature, and is, for example, 3 minutes or more, 10 minutes or more, even 30 minutes or more, or, in some cases, 1 hour or more. The upper limit of the heating time is not limited to a particular time, and may be 10 hours or less in terms of mass production. A water vapor treatment is effective to improve the water repellency of the water-repellent film. Although the details of the reason are unknown, the water repellency of the water-repellent film can be improved by a water vapor treatment. The water vapor treatment may be performed by supplying heated water vapor to the water-repellent film. The temperature of the water vapor is, for example, but not particularly limited to, 80°C or higher, or even 90°C or higher. The time during which the film is subjected to the water vapor treatment is not limited to a particular time, either, and is, for example, 30 seconds or more, or even 1 minute or more.

### <Applications>

### (Cosmetic preparations)

The flaky-shaped substrate with the water-repellent film according to the present embodiment is particularly suitable for use as a material of cosmetic preparations. The cosmetic preparations are, for example, but not particularly limited to, facial cosmetics, makeup cosmetics, and hair cosmetics. Cosmetic preparations in which the flaky-shaped substrate with the water-repellent film according to the present embodiment is particularly preferably included are facial cosmetics, such as foundations and face powders. The cosmetic preparations are not limited to particular forms, and examples include powders, cakes, pencils, sticks, ointments, liquids, emulsions, and creams.

### (Other Applications)

The flaky-shaped substrate with the water-repellent film according to the present embodiment may be used in other applications than the cosmetic preparations. For example, the flaky-shaped substrate with the water-repellent film according to the present embodiment may be included in compositions to be applied, such as paints and inks, or may be included in resin molded articles, such as resin parts and resin containers. The flaky-shaped substrate with the water-repellent film according to the present embodiment is also suitably included in compositions or shaped articles used for painting or parts of products that are, for example, for outdoor use and desirably imparted with a water-repellent effect.

Hereinafter, the flaky-shaped substrate will be described in more detail by examples. The examples given below are not offered to limit the present invention, either.

### (Example 1)

An amount of 7.58 g of an aqueous diacetoxy zirconium oxide solution (Tokyo Chemical Industry Co., Ltd.) and 686.2 g of an ethanol-based solvent (Fineeter A-10 by FUTABA PURE CHEMICAL CO., LTD.) were added to 10.20 g of cerium nitrate hexahydrate (FUJIFILM Wako Pure Chemical Corporation), and the mixture was stirred at 40°C for 18 hours. After the stirring, 96 g of glass flakes (FTD010FY-F01 by Nippon Sheet Glass Co., Ltd.) having an average particle diameter of 10 µm and a thickness of 0.4 µm were added.

A spray-drying method was applied to the dispersion liquid of the glass flakes. For the spray-drying method, a spray dryer (TR160 with a two-fluid nozzle) was used under conditions of an inlet temperature of 80°C and an outlet temperature of 67 to 43°C. Coated glass flakes obtained by the spray-drying method were held in a furnace at 460°C for four minutes to be fired. Approximately 1 g of the fired glass flakes were put in a beaker, which was then covered with an aluminum foil. A hole was made in the aluminum foil, and a nozzle of a steam cleaner (STM-304 by Iris Ohyama Inc.) was inserted in the hole. A water vapor treatment was performed in which water vapor at approximately 100°C was supplied to the glass flakes for approximately three minutes. After that, the glass flakes were dried at 120°C for approximately two hours. Glass flakes each coated with a water-repellent film including cerium oxide and zirconium oxide were obtained in this manner.

### (Example 2)

An amount of 0.95 g of titanium lactate (ORGATIX TC-315 by Matsumoto Fine Chemical Co., Ltd.), 84.6 g of purified water, and 12 g of glass flakes (FTD010FY-F01 by Nippon Sheet Glass Co., Ltd.) were added to 2.45 g of cerium nitrate hexahydrate (FUJIFILM Wako Pure Chemical Corporation), and the mixture was stirred at 40°C for 18 hours. After that, the liquid temperature was decreased to room temperature.

Vacuum filtration was applied to the dispersion liquid of the glass flakes. The vacuum filtration was performed at a suction pressure of 0.08 MPa for a filtration time of 15 minutes using filter paper 5C (diameter: 110 mm; particle retention size: 1 µm) manufactured by Advantec Toyo Kaisha, Ltd. Coated glass flakes obtained by the vacuum filtration were held in a furnace at 650°C for one hour to be fired. After that, a water vapor treatment and drying were performed in the same manner as in Example 1. Glass flakes each coated with a water-repellent film including cerium oxide and titanium oxide were obtained in this manner.

### (Example 3)

An amount of 1.82 g of basic aluminum lactate (Takiceram M-160L by Taki Chemical Co., Ltd.), 84.22 g of purified water, and 12 g of glass flakes (FTD010FY-F01 by Nippon Sheet Glass Co., Ltd.) were added to 1.96 g of cerium nitrate hexahydrate (FUJIFILM Wako Pure Chemical Corporation), and the mixture was stirred at 40°C for 18 hours. After that, the liquid temperature was decreased to room temperature. After that, glass flakes each coated with a water-repellent film including cerium oxide and aluminum oxide were obtained in the same manner as in Example 2.

### (Example 4)

An amount of 697.1 g of an ethanol-based solvent (FineeterA-10 by FUTABA PURE CHEMICAL CO., LTD.) was added to 6.89 g of cerium nitrate hexahydrate (FUJIFILM Wako Pure Chemical Corporation), and the mixture was stirred at 40°C for 18 hours. After the stirring, 96 g of glass flakes (FTD010FY-F01 by Nippon Sheet Glass Co., Ltd.) were added, and the mixture was stirred at room temperature for approximately one hour. After that, glass flakes each coated with a water-repellent film including cerium oxide were obtained in the same manner as in Example 1.

### (Example 5)

Glass flakes each coated with a water-repellent film including cerium oxide were obtained in the same manner as in Example 1, except that the water vapor treatment was omitted.

### (Float Test)

An amount of 0.1 g of the water-repellent film-attached glass flakes obtained in each Example was put into 5 g of water at room temperature, and was left to stand still for three weeks with the water held at room temperature. The water repellency thereof was evaluated by visual observation according to the following criteria.
A: Every water-repellent film-attached flaky substrate remained floating on or in the water three weeks after being put into the water.
B: On a mass basis, 75% or more of the water-repellent film-attached flaky substrates remained floating on or in the water three weeks after being put into the water.
C: On a mass basis, 25 to 75% of the water-repellent film-attached flaky substrates remained floating on or in the water three weeks after being put into the water.
D: On a mass basis, 75% or more of the water-repellent film-attached flaky substrates settled within three weeks or immediately after being put into the water.

Table 1 shows the results for Examples along with the types of oxides included in the water-repellent films, the molar ratios, and the heating conditions.

**[Table 1]**

| Ex. | Rare-earth oxide M1 | Oxide M2 other than rare earth | M1/M2 (molar ratio) | Heating conditions | Water vapor treatment | Float test |
|---|---|---|---|---|---|---|
| 1 | Cerium oxide | Zirconium oxide | 3.5 | 460°C/4 min | Applied | A |
| 2 | Cerium oxide | Titanium oxide | 3.5 | 650°C/1 h | Applied | A |
| 3 | Cerium oxide | Aluminum oxide | 3.5 | 650°C/1 h | Applied | A |
| 4 | Cerium oxide | - | - | 460°C/4 min | Applied | B |
| 5 | Cerium oxide | Zirconium oxide | 3.5 | 460°C/4 min | Not applied | D |

In Example 4, approximately 80 mass% of the water-repellent film-attached glass flakes were floating at the end of the float test. They were collected to separate from the remaining portion that had settled. The above float test was performed again for the collected water-repellent film-attached glass flakes, which were rated A. It should be noted that although the float test result for Example 5 is D, the water-repellent films of Example 5 had higher water-repellent properties than the surfaces of the glass flakes, namely, the glass surfaces.

MIU and MMD were measured for Example 1; MIU was 2.9, and MMD was 0.25. The measurement was performed using a friction tester (KES-SE by KATO TECH CO., LTD.) under conditions of a static load of 50 g and a speed of 1 mm/sec. The friction head used was a 10 mm-square silicone element. A measurement specimen was produced by uniformly applying the powder specimen to black artificial leather (protein leather (registered trademark) SUPPLALE by IDEATEX JAPAN Co., Ltd.) so that the mass per unit area was 1.3 mg/cm².

## Claims

1. A flaky-shaped substrate with a water-repellent film, the flaky-shaped substrate comprising a flaky substrate and a water-repellent film formed on the flaky substrate, wherein
the water-repellent film includes a rare-earth oxide.

2. The flaky-shaped substrate with the water-repellent film according to claim 1, having enough water repellency to float on water at room temperature for three weeks.

3. The flaky-shaped substrate with the water-repellent film according to claim 1, wherein the rare-earth oxide includes at least one selected from the group consisting of cerium oxide, lanthanum oxide, yttrium oxide, praseodymium oxide, and erbium oxide.

4. The flaky-shaped substrate with the water-repellent film according to claim 1, wherein the water-repellent film further includes at least one selected from the group consisting of aluminum oxide, zirconium oxide, titanium oxide, zinc oxide, and silicon oxide.

5. The flaky-shaped substrate with the water-repellent film according to claim 1, wherein the water-repellent film consists substantially of an inorganic substance.

6. The flaky-shaped substrate with the water-repellent film according to claim 1, wherein the flaky substrate consists substantially of an inorganic substance.

7. The flaky-shaped substrate with the water-repellent film according to claim 1, wherein the water-repellent film includes the rare-earth oxide as a main component.

8. The flaky-shaped substrate with the water-repellent film according to claim 1, further comprising an intermediate film between the flaky substrate and the water-repellent film.

9. A composition comprising the flaky-shaped substrate with the water-repellent film according to any one of claims 1 to 8 and a resin.

10. A cosmetic preparation comprising the flaky-shaped substrate with the water-repellent film according to any one of claims 1 to 8.

11. A painted body comprising: a paint film including the flaky-shaped substrate with the water-repellent film according to any one of claims 1 to 8; and a support supporting the paint film.
